# EUROPÄISCHE PATENTSCHRIFT

(11) **EP 3 346 942 B1**
(45) Veröffentlichungstag und Bekanntmachung des Hinweises auf die Patenterteilung: **05.02.2020**
(21) Anmeldenummer: 16760733.2
(22) Anmeldetag: 06.09.2016
(51) Int. Cl.: A61C 11/00, A61C 19/045

(54) **VERFAHREN ZUR BEHANDLUNGSPLANUNG**
METHOD FOR TREATMENT PLANNING
PROCÉDÉ DE PLANIFICATION DE TRAITEMENT

(30) Priorität: 08.09.2015 DE 102015115034
(43) Veröffentlichungstag der Anmeldung: 18.07.2018
(73) Patentinhaber: Sicat GmbH & CO. KG, 53177 Bonn (DE)
(72) Erfinder: LEHNER, Tobias, 53229 Bonn (DE); KUSCH, Jochen, 53343 Wachtberg (DE); HANSSEN, Nils, 53125 Bonn (DE)
(74) Vertreter: Braun-Dullaeus Pannen Emmerling Patent- & Rechtsanwaltspartnerschaft mbB
(86) Internationale Anmeldenummer: PCT/EP2016/070965
(87) Internationale Veröffentlichungsnummer: WO 2017/042156

(56) Entgegenhaltungen:
- DE-A1-102010 021 934
- US-A1- 2010 152 873
- US-A1- 2015 132 716

## Beschreibung

Die Erfindung betrifft ein Verfahren und ein System zur Planung einer Behandlung am Gebiss eines Patienten, wobei der Planung eine Darstellung einer Kaubewegung des Patienten auf einem Computerbildschirm zugrunde liegt, bei der sich die obere und die untere Zahnreihe gegeneinander bewegen.

Bildgebende Verfahren und elektronische Registrierungen zählen zu den wichtigsten Methoden für die Erfassung des Funktionszustandes des stomatognathen Systems und für Planungen komplexer prothetischer Restaurationen. Dabei sind verschiedene Verfahren bekannt, anatomische Verhältnisse im lebenden Körper zu visualisieren, um einem behandelnden Arzt die Diagnose zu erleichtern und eine optimierte Planung der Therapie zu ermöglichen. Beispielsweise ist aus der DE 10 2012 104 912 A1 die anatomische und vor allem auch die funktionale kinematische Darstellung von Kiefergelenken in volumetrischen respektive in Oberflächen-Ansichten in drei Dimensionen bekannt. Dabei kann ein digitales volumetrisches Unterkieferabbild in unterschiedlichen Stellungen gegenüber einem digitalen volumetrischen Oberkieferabbild dargestellt werden. Mittels Kondylographie wird eine Bewegung aufgezeichnet und eine Vielzahl von Positionsdatensätzen gespeichert ("Kondylogramm"). Ein solcher Positionsdatensatz beschreibt die reale räumliche Stellung des Unterkiefers gegenüber dem Oberkiefer an einem bestimmten Punkt der Bewegung. Bei dem Verfahren werden die Datensätze auf der Grundlage eines ersten, mit einem volumentomographischen Verfahren aufgenommenen Datensatzes unter Berücksichtigung der ermittelten und im Kondylogramm enthaltenen Bewegungsdaten rechnerisch "simuliert" und dem Betrachter auf dem Bildschirm dargestellt.

Ein solches Verfahren ist auch aus dem Artikel "SICAT Function: Anatomical Real-Dynamic Articulation by Merging Cone Beam Computed Tomography and Jaw Motion Tracking Data", International Journal of Computerized Dentistry 2014, 17(1); 65-74, bekannt. Eine zentrale Rolle bei der offenbarten Verfahrensweise spielt eine Bissschablone ("FusionBite"), die Zahnimpressionen des Patienten, mithin Abdrücke der beiden Zahnreihen, in einer ausgehärteten Masse aufweist. Mit dieser Bissschablone ist die exakte räumlich Zuordnung zwischen 3D-Röntgendaten und den aufgenommenen (Kau-) Bewegungsdaten möglich. Die Bissschablone wird vom Patienten während einer Röntgenaufnahme getragen. Nachdem die Daten mit Hilfe der Bissschablone räumlich exakt zugeordnet wurden, kann die Bewegung der Kiefergelenke mittels eines Computerprogrammes nachvollzogen werden. Die simulierte Bewegung der digitalen Zahnabdrücke lässt sich wiederum auf einem Bildschirm darstellen und an der bewegten Darstellung exakt untersuchen.

Bei den bekannten Verfahren werden die volumentomographischen Daten mittels Röntgenscan am Patienten aufgenommen, der während der Untersuchung einer entsprechenden Strahlenbelastung ausgesetzt ist. Bei vielen Röntgengeräten ist der Aufnahmebereich jedoch zu klein, um in der Aufnahme die Kiefergelenke sehen zu können. Der Aufnahmebereich solch kleiner Röntgengeräte reicht jedoch aus, um sie für die oben beschriebene Verfahrensweise zu verwenden und die patientenindividuelle Bewegung der Zahnreihen anhand der digitalen Zahnabdrücke zu simulieren. Auch bei solchen Röntgengeräten wird der Patient der ionisierenden Strahlung ausgesetzt, nur um die räumliche Zuordnung über die Bissschablone herstellen zu können.

Aus der DE 10 2013 204 207 A1 ist eine Bissgabel bekannt, mit der eine Relation zwischen den Messdaten eines intraloralen 3D Oberflächenscanners und den Daten eines 3D Positioniersystems hergestellt und der Zahnbogen erfasst werden kann, um die Oberflächen statisch und in Bewegung darstellen zu können.

Aus der DE 10 2010 021 934 A1 ist ein ähnliches Dental-Werkzeug zur Gewinnung von Korrelationsdaten bekannt, das mit einer an einem Stirnbogen gehaltenen Sensorik zusammenwirkt, um Bewegungsmessungen durchführen zu können.

US 2015/0132716 A1 zeigt eine Methode, um ein virtuelles Kieferbild zu erzeugen, wobei die Methode beinhaltet, dass eine Bewegungsaufnahme gemacht wird, welche zumindest einen Satz von Positionsdaten an einem definierten Zeitpunkt aufweist.

US 2010/0152873 A1 zeigt eine Methode, um aus einer Kombination von digitalen dreidimensionalen Modellierungen und schnellen Herstellungsmethoden Komponenten für Zahnmodelle bereitzustellen.

Aufgabe der vorliegenden Erfindung ist es nunmehr, ein mit einfachen Mitteln umzusetzendes Verfahren zur Darstellung der Kaubewegung zu schaffen, das die Strahlenbelastung des Patienten minimiert und an dem sich anatomische Besonderheiten einfach feststellen und Planungen prothetischer Restauration unter Berücksichtigung patientenindividueller Kaugewohnheiten vornehmen lassen.

Diese Aufgabe wird durch das Verfahren mit den kennzeichnenden Merkmalen des Anspruch 1 und das System nach Anspruch 9 gelöst. Vorteilhafte Ausführungsformen sind in den Unteransprüchen genannt.

Entsprechend Anspruch 1 liegt der Kern der Erfindung darin, vollständig auf den Röntgenscan am Patienten zu verzichten und einen solchen, der schließlich zur Registrierung der Datensätze, mithin zur deren exakten Fusionierung, nötig ist an einem Modell durchzuführen, das den Biss des Patienten repräsentiert. Erfindungsgemäß wird dem Patienten dazu eine Bissschablone der bekannten Art angepasst, die ausgehärtete Impressionen des zumindest teilweise bezahnten Oberkiefers und des zumindest teilweise bezahnten Unterkiefers aufweist. Um die Orientierung der Bissschablone im zweiten Datensatz der gescannten Daten besser nachvollziehen zu können, ist diese mit identifizierbaren Markern versehen. Der zweite Datensatz enthaltend dreidimensionale Bilddaten der Bissschablone wird dann ohne Patientenbeteiligung aufgenommen. Dieser zweite Datensatz ist so aufzunehmen, dass sich die Oberflächen der bezahnten Kiefer und die Marker der Bissschablone identifizieren lassen.

Erfindungsgemäß gibt es nun zwei Möglichkeiten den zweiten Datensatz aufzunehmen. Zum einen ist es möglich, nur die Bissschablone mit der daran befindlichen ausgehärteten Abdruckmasse, in die sich die Zahnimpressionen eingegraben haben, aufzunehmen. Aus den "negativen" Zahnimpressionen lassen sich dann nachträglich die "positiven" Oberflächen der bezahnten Kiefer errechnen. Falls nur die Bissschablone mit einem 3D-Röntgenverfahren gescannt wird, ist es vorteilhaft, eine radioopake Abdruckmasse zu verwenden, um die (Negativ-)Kontur der Zähne besser erkennen zu können.

In einer vorteilhaften Ausführungsform weist die Bissschablone im vorderen Bereich einen Steckadapter ("Flansch") auf, über den eine starre mechanischen Verbindung mit dem Aufnahmesystem zur Erstellung eines zweiten Datensatzes, insbesondere mit dem Röntgengerät, möglich ist.

Auf der anderen Seite ist es möglich, den zweiten Datensatz von einem Modell des Gebisses, insbesondere einem Gipsmodell, mit Ober- und Unterkiefer des Patienten anzufertigen. Bei dem Modell liegen die Oberflächen der bezahnten Kiefer als "positive" Oberflächen vor. Während des Scans liegen die Gipsmodelle formschlüssig in den Impressionen der Bissschablone.

Der an der Bissschablone anzufertigende Scan wird mit einem Verfahren vorgenommen, das es erlaubt, die negativen und/oder positiven Oberflächen aufzulösen. Unter Umständen ist das mit einem optischen Kamerascan möglich, einen dreidimensionalen Datensatz aufzunehmen. Vorteilhaft ist es jedoch, wenn der Scan mit der Röntgenstrahlung eines Digitalen Volumen Tomographen (DVT) aufgenommen wird. Mit einem solchen lassen sich die Oberflächen der Bissschablone und/oder des Modells exakt darstellen. Bei Einsatz von Röntgenstrahlung ist es vorteilhaft, die Marker an der Bissschablone aus einem radioopaken Material, beispielsweise in Form kleiner eingebrachter Kügelchen, auszuführen, um sie in dem dreidimensionalen Datensatz gut identifizieren zu können.

Im Einzelnen manifestiert sich das Verfahren zur Planung einer Behandlung am Gebiss eines Patienten auf der Grundlage der Darstellung einer Kaubewegung von oberer und unterer Zahnreihe des Patienten in folgenden Schritten:
Zunächst wird mit einem intraoralen Kamerasystem ein erster Datensatz mit dreidimensionalen Bilddaten der Oberflächen des mehr oder weniger bezahnten Oberkiefers und des mehr oder weniger bezahnten Unterkiefers aufgenommen. Während und nach der Aufnahme lassen sich die Kiefer anhand der Daten auf einem Bildschirm visualisieren. Das Verfahren funktioniert auch mit teilbezahnten Kiefern. Es müssen lediglich so viele Zähne vorhanden sein, dass eine Registrierung durchgeführt werden kann.

Zwischenzeitlich wird dem Patienten eine Bissschablone ("FusionBite") angepasst, indem er in die darauf aufgebrachte Abdruckmasse beißt. Nach dem Aushärten weist die Bissschablone die ausgehärtete Impressionen des bezahnten Oberkiefers und des bezahnten Unterkiefers auf. Um im späteren Scan sichtbar zu sein, sind an und/oder in der Bissschablone identifizierbare Marker definierter Gestalt vorgesehen, die zur Sichtbarkein im Röntgenscan aus einem radioopaken Material bestehen. Die Marker stehen in einem bekannten räumlichen Verhältnis zum Steckadapter des FusionBites um eine räumlich exakte Registrierung mit den Bewegungsdaten zu ermöglichen.

Erfindungsgemäß wird dann ohne jegliche Patientenbeteiligung der zweite Datensatz mit dreidimensionalen Bilddaten enthaltend Oberflächendaten der Bissschablone und gegebenenfalls eines Modells aufgenommen. Die Aufnahmetechnik ist so zu wählen, dass sich in den Daten die Oberflächen der bezahnten Kiefer und die Marker der Bissschablone identifizieren lassen.

Für die Aufnahme der dreidimensionalen Bilddaten mit Oberflächendaten der Bissschablone und des Modells ist es vorteilhaft, wenn die Bissschablone von einem insbesondere zylindrischen Metallblech unterschiedlicher Dicke, beispielsweise aus Kupfer oder Aluminium, umgeben ist. Das Metallblech kann mit der Bissschablone über den Adapter verbunden werden. Es bewirkt eine Röntgenabschwächung, die dem Weichgewebe des (fehlenden) Patienten ähnlich ist. Damit weisen die Röntgenaufnahmen ähnliche Grauwert-Charakeristika wie ein echter Patientenscan auf.

Zur Aufnahme der Bewegungsdaten wird am Unterkiefer des Patienten ein beweglicher lösbar befestigt. Um eine Registrierung der Lage des Bewegungsaufnehmers gegenüber der Bissschablone vornehmen zu können, findet die Befestigung statt, während der Patient auf die angepasste Bissschablone beißt. Nach der Registrierung wird die Bissschablone dem Mund des Patienten entnommen. Als Gegenstück zu dem Bewegungsaufnehmer dient ein (Bewegungs)-Detektor, der in fester Position am Kopf des Patienten befestigt wird und aus dieser Position heraus die Bewegungen des Bewegungsaufnehmers registriert. Mit dem lösbar fixierten Bewegungsaufnehmer und dem lösbar fixierten Detektor vollzieht der Patient nun eine Abfolge mehrerer Kaubewegungen möglichst auf unterschiedlichen Bahnen soweit es die Anatomie seines Kiefers zulässt. Diese Kaubewegungen werden mit dem Detektor aufgenommen und in einem dritten Datensatz als Bewegungsdaten gespeichert. Die Bewegungsdaten repräsentieren Bewegungslinien jeweils einzelner, mit dem Unterkiefer in Beziehung stehender Punkte in einem durch den Oberkiefer fixierten dreidimensionalen Koordinatensystem.

Schließlich werden die Bewegungsdaten mit den dreidimensionalen Bilddaten des ersten Datensatzes in ein gemeinsames Koordinatensystem gebracht ("registriert"), so dass an den dreidimensionalen Bilddaten von oberer und unterer Zahnreihe deren Bewegung simuliert werden kann. Dabei werden die dreidimensionalen Bilddaten des ersten Datensatzes mit den patientenindividuellen Bewegungsdaten des dritten Datensatzes vermittels der Registrierung über den zweiten Datensatzes verheiratet.

Letztendlich können die Kaubewegungen des bezahnten Unterkiefers gegenüber dem bezahnten Oberkiefer auf einem Bildschirm zum Zwecke der Behandlungsplanung dargestellt werden. Im Rahmen der Behandlungsplanung kann der behandelnde Arzt virtuell ein Implantat in einen der Kiefer einsetzen und beobachten, wie es sich in die Anatomie der simulierten Kaubewegung einpasst.

Das erfindungsgemäße Verfahren und die erfindungsgemäße Vorrichtung gewährleisten über die direkte Fusion von digitalen Zahnmodellen und Bewegungsaufnahmen eine praxistaugliche und zugleich präzise Lösung. Auf diese Weise gelingt es, die echte patientenindividuelle Zahnreihenposition und die Bewegung der Zahnreihen im 3D-Volumen anatomiegetreu darzustellen. Dabei können digitale Modelle des Ober- und Unterkiefers, die mit intraoralen oder laborseitigen Scanverfahren gewonnen wurden, mit den DVT-Daten überlagert werden, womit die Voraussetzung geschaffen wurde, therapeutische Aufbissbehelfe oder Zahnersatz in optimierter Kieferrelation anzufertigen und deren Design den patientenindividuellen Bewegungsmustern zu unterwerfen.

Der besondere Vorteil der erfindungsgemäßen Vorgehensweise, die sich auf die simulierende Darstellung der Kaubewegungen von Ober- und Unterkiefer bezieht, liegt darin, dass dem Patienten die Strahlenbelastung durch ionisierende Strahlung erspart bleibt. Die räumliche Zuordnung zwischen dem ersten und dem dritten Datensatz geschieht erfindungsgemäß über den am Modell und nicht am Patienten aufgenommenen zweiten Datensatz.

Zur Bewegungsmessung und Bewegungsregistrierung kommt vorteilhafterweise ein System umfassend einen Bewegungsaufnehmer und einen Detektor zum Einsatz, das z.B. auf Ultraschalllaufzeitbasis, also einer Umrechnung von Laufzeiten mehrerer akustischer Signale in Rauminformationen, arbeitet. Am Patienten wird dazu paraokklusal eine Halterung ("Attachment") für den Bewegungsaufnehmer lösbar befestigt, der in diesem Fall ein mit Schallsendern bestückter Messsensorbogen ist. Der Bewegungsaufnehmer besitzt eine magnetische Kopplung, mit der er am Unterkieferattachment und an der Bissschablone stabil befestigt werden kann. Mittels des Bewegungsaufnehmers werden vorteilhafterweise vier Ultraschallsender am Unterkiefer breitflächig vor dem Mund und zu den Seiten in Bogenform angeordnet. Der oberhalb befestigte Detektor besteht aus jeweils links und rechts angeordneten drei kollinear positionierten Mikrofonen, sodass insgesamt ein gleichzeitig okklusionsnahes als auch gelenknahes Messfeld definiert wird.

Als Gegenpart zum mit dem Unterkiefer beweglichen Messsensorbogen umfasst das System eine am Kopf fixierte Mikrofon-Sensor-Einheit, die an der Glabella und an dem Mastoid postauricular beidseits angelegt und durch ein Gummiband über den Hinterkopf fixiert wird. Damit erfolgt ganz natürlich während der Kaubewegungen eine Bewegungsmessung nur über die zum Kopf relative Kaubewegung des Unterkiefers.

Für die Referenzierung mit dem zweiten Datensatz wird die Bissschablone interponiert, der Messensorbogen an der Bisschablone platziert und für wenige Sekunden eine Messung ausgelöst. Anschließend wird die Bissschablone entfernt, der Messensorbogen am Attachment befestigt und die eigentliche Funktionsuntersuchung durchgeführt.

Nach der Fusionierung des zweiten Datensatzes und der Bewegungsdaten stehen dem Benutzer alle gemessenen Kieferbewegungen und -positionen zur Verfügung. Anhand einer Auswahlliste kann zwischen verschiedenen Kieferstellungen ausgewählt werden. Wird eine Bewegung aus der Liste ausgewählt, wird automatisch eine Bewegungsspur mit anatomischem Bezug angezeigt.

Die Erfindung wird nachfolgend anhand der Figuren 1 bis 3 näher beschrieben. Es zeigen:
- **Figur 1 (a, b)**: eine Bissschablone
- **Figur 2 (a, b)**: das System zur Aufnahme von Bewegungsdaten und
- **Figur 3 (a-c)**: die Simulation der Kaubewegung an Bilddaten.

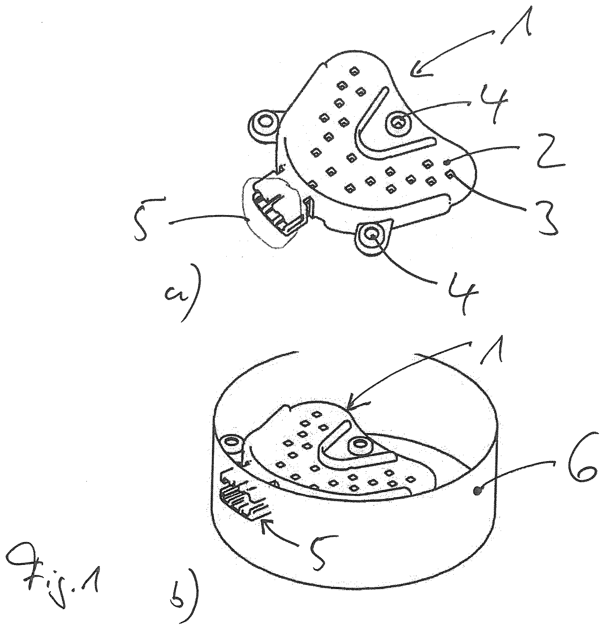

Figur 1a zeigt eine Bissschablone 1, die in diesem Stadium noch nicht mit Abdruckmasse bestückt ist. Diese wird beidseitig auf die jeweilige Auflagefläche 2 aufgebracht, bevor der Patient seinen Biss in der auszuhärtenden Abdruckmasse hinterlässt. Die Abdruckmasse hält vermittels Verzahnungen 3, die in die Auflagefläche 2 eingebracht sind. An der Bissschablone 1 sind Marker 4 aus einem radioopaken Material angebracht, die sich im späteren Röntgenscan identifizieren lassen. Die Bissschablone 1 weist zudem einen Steckadapter 5 auf, über den sie an dem Aufnahmegerät fixiert werden kann. In Figur 1b ist die Bissschablone 1 von einem Zylinder aus dünnem Blech 6 umgeben, der die Röntgenstrahlung beim Röntgenscan ähnlich wie das den Kiefer umgebende Gewebe eines realen Patienten teilweise absorbiert.

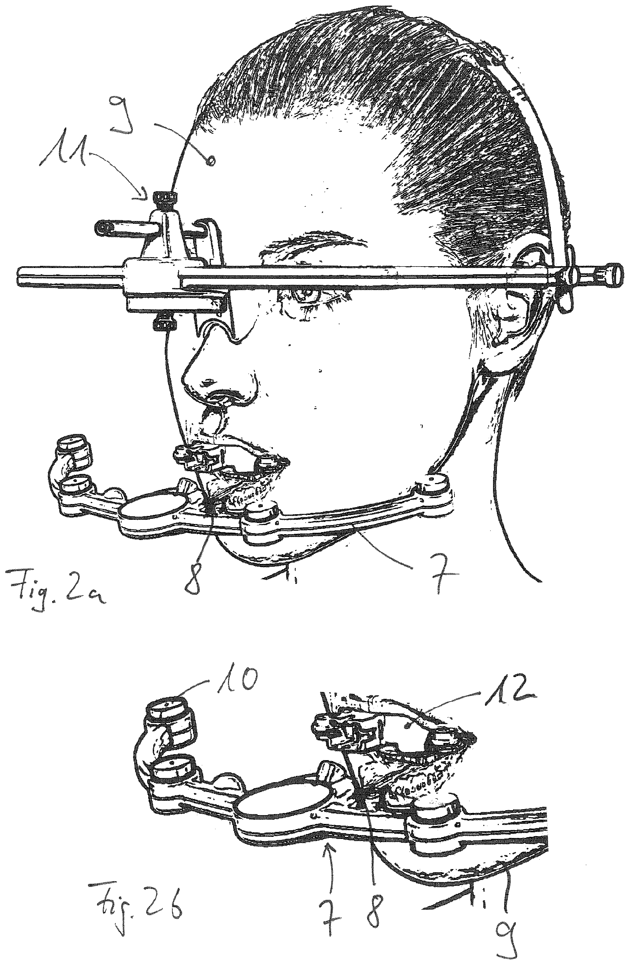

Figur 2a zeigt einen Bewegungsaufnehmer 7 in Bogenform, der, wie aus der Detailansicht nach Figur 2b ersichtlich, über einen Halter 8 ("Attachment") am Unterkiefer einer Patientin 9 lösbar befestigt ist. Auf dem Bewegungsaufnehmer 7 sind vier Ultraschallsender 10 zu beiden Seiten angeordnet. Der oberhalb an der Stirn der Patientin befestigte Detektor 11 hat drei jeweils links und rechts angeordnete kollinear positionierte Mikrofone, die damit das Messfeld definieren. Im vorliegenden Fall trägt die Patientin 9 zusätzlich die Bissschablone 12. In diesem Zustand erfolgt die Messung zur Registrierung des über den Halter 8 angebrachten Bewegungsaufnehmers 7, der kurz zuvor über einen Adapter an der Bissschablone 12 befestigt war.

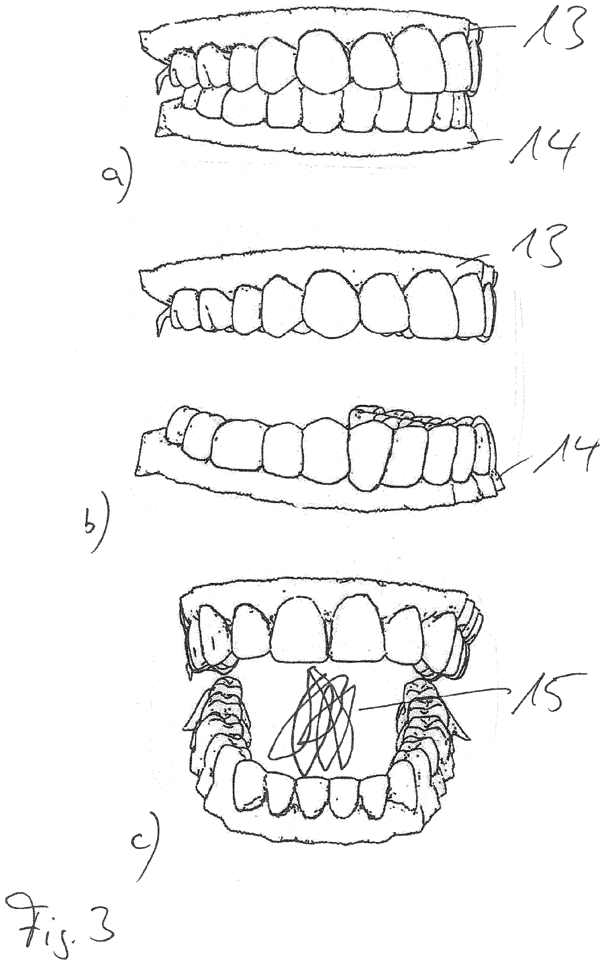

Die Figuren 3 zeigen nunmehr das Ergebnis der erfindungsgemäßen Verfahrensweise, nämlich die auf einem Bildschirm dargestellten Zahnreihen von Oberkiefer 13 und Unterkiefer 14 in verschiedenen simulierten Bewegungsstadien und aus verschiedenen Perspektiven. In Figur 3a ist das Gebiss vollständig geschlossen und in Figur 3b maximal geöffnet. Figur 3c ist eine Frontalansicht auf das geöffnete Gebiss. In den offenen Mundraum sind die Bewegungsspuren 15 während verschiedener Kaubewegungen eingezeichnet. Der behandelnde Arzt kann nun an dem virtuellen Gebiss z.B. prothetische Restaurationen planen.

Nachfolgend sind noch einmal zusammenfassend die einzelnen Schritte hin zu dieser Simulation dargestellt, wobei die Abfolge der Schritte nicht auf diese festgelegt ist:
- Schritt 1:: Aufnahme der digitalen Zahnabdrücke **(erster Datensatz);**
- Schritt 2:: Beschicken der Bissschablone mit der aushärtenden Abdruckmasse;
- Schritt 3:: Erstellen eines zweiteiligen Gipsmodells auf der Grundlage der Abformung;
- Schritt 4:: Erstellen des 3D-Röntgenscan **(zweiter Datensatz)** mit der Bissschablone, die von den Kiefern des Gipsmodelles gehalten wird;
- Schritt 5:: Wiedereinsetzen der Bissschablone in den Mund des Patienten zum Kalibrieren des Bewegungsaufzeichnugsgerätes;
- Schritt 6:: Befestigung des T-Attachments an die Unterkiefer-Zähne des Patienten. Mit dem T-Attachment findet - nachdem die Kalibrierung des Gerätes durchgeführt wurde - die eigentliche Bewegungsmessung statt;
- Schritt 7:: Initiale Kalibrierung des Gerätes zur Bewegungsmessung mit der Bissschablone;
- Schritt 8:: Barrierefreie Bewegungsmessung **(dritter Datensatz)** mit dem Bewegungsaufzeichnugsgerätes ohne Bissschablone;
- Schritt 9:: Registrierung der 3D-Röntgendaten **(zweiter Datensatz)** und der Bewegungsdaten **(dritter Datensatz)** mit Hilfe der radioopaken Kugelmarker der Bissschablone;
- Schritt 10:: Registrierung der digitalen Zahnabdrücke **(erster Datensatz)** mit den 3D-Röntgendaten **(zweiter Datensatz);**
- Schritt 11:: Bewegung der digitalen Zahnabdrücke **(erster Datensatz)** und des Unterkiefers anhand der patientenindividuellen Bewegungen **(dritter Datensatz).**

Erfindungsgemäß werden die Daten des zweiten Datensatzes nur benötigt, um die Daten des ersten Datensatzes und die Daten des dritten Datensatzes mittelbar über die Daten des zweiten Datensatzes in räumlichen Einklang zu bringen:

## Patentansprüche

1. Verfahren zur Planung einer Behandlung am Gebiss eines Patienten (9), wobei der Planung eine Darstellung einer Kaubewegung des Patienten (9) zugrunde liegt, bei der sich die obere und die untere Zahnreihe gegeneinander bewegen,
**dadurch gekennzeichnet,**
**dass** mit einer Kamera ein erster Datensatz mit dreidimensionalen Bilddaten der Oberflächen des zumindest teilweise bezahnten Oberkiefers (13) und des zumindest teilweise bezahnten Unterkiefers (14) aufgenommen werden,
**dass** dem Patienten (9) eine Bissschablone (1) angepasst wird, die ausgehärtete Impressionen des Oberkiefers (13) und des Unterkiefers (14) aufweist, wobei an und/oder in der Bissschablone (1) identifizierbare Marker (4) vorgesehen sind,
**dass** ohne Patientenbeteiligung ein zweiter Datensatz mit dreidimensionalen Bilddaten enthaltend Oberflächendaten der Bissschablone (1) aufgenommen wird, in denen sich die Oberflächen der Kiefer (13,14) und die Marker (4) der Bissschablone (1) identifizieren lassen,
**dass** am Unterkiefer (14) des Patienten (9) ein Bewegungsaufnehmer (7) lösbar befestigt wird, während er auf die angepasste Bissschablone (1) beißt, wobei die Lage des Bewegungsaufnehmers (7) gegenüber der Bissschablone (1) registriert ist,
**dass** die Bissschablone entnommen wird,
**dass** die Bewegungen des Bewegungsaufnehmers (7) bei Kaubewegungen mit einem am Kopf des Patienten (9) lösbar befestigten Detektor (11) aufgenommen und in einem dritten Datensatz als Bewegungsdaten gespeichert werden,
**dass** die dreidimensionalen Bilddaten des ersten Datensatzes mit den Bewegungsdaten des dritten Datensatzes vereinigt werden, wobei eine Registrierung vermittels des zweiten Datensatzes geschieht,
**dass** die Kaubewegungen des Unterkiefers (14) gegenüber dem Oberkiefer (13) auf einem Bildschirm zum Zwecke der Behandlungsplanung dargestellt werden.

2. Verfahren nach Anspruch 1,
**dadurch gekennzeichnet, dass** der zweite Datensatz allein von der Bissschablone (1) aufgenommen wird.

3. Verfahren nach Anspruch 1,
**dadurch gekennzeichnet, dass** der zweite Datensatz von einem mit der Bissschablone (1) bestückten Modell, insbesondere einem Gipsmodell, mit Ober- und Unterkiefer (13,14) des Patienten angefertigt wird, wobei zwischen dem Ober- und dem Unterkiefer des Modells die Bissschablone (1) eingesetzt wird.

4. Verfahren nach einem der vorherigen Ansprüche,
**dadurch gekennzeichnet, dass** der zweite Datensatz von der Bissschablone (1) oder dem mit Bissschablone (1) bestückten Modell mit einem dreidimensionalen Röntgenscan aufgenommen wird.

5. Verfahren nach einem der vorherigen Ansprüche,
**dadurch gekennzeichnet, dass** eine Halterung (8) zur lösbaren Befestigung des Bewegungsaufnehmers (7) vorgesehen ist, die am Unterkiefer des Patienten durch Aufkleben fixiert wird.

6. Verfahren nach Anspruch 5,
**dadurch gekennzeichnet, dass** der Bewegungsaufnehmer (7) zunächst an einer Halterung (5) der Bissschablone (1) fixiert und diese erste Lage des Bewegungsaufnehmers (7) mit dem Detektor (11) aufgenommen wird, wobei nachfolgend der Bewegungsaufnehmer (7) an der aufgeklebten Halterung (8) fixiert und diese zweite Lage des Bewegungsaufnehmers (7) mit eingesetzter Bissschablone (1) mit dem Detektor (11) aufgenommen wird, wobei nachfolgend die Bissschablone (1) entnommen wird, um ungestörte Kaubewegungen zu ermöglichen

7. Verfahren nach einem der vorherigen Ansprüche,
**dadurch gekennzeichnet, dass** die Vereinigung der dreidimensionalen Bilddaten des ersten Datensatzes mit den Bewegungsdaten des dritten Datensatzes derart geschieht, dass zunächst eine Registrierung der Bewegungsdaten und der Daten des zweiten Datensatzes mittels der darin erkennbaren Marker (4) der Bissschablone (1) vorgenommen wird und dass nachfolgend eine Registrierung der Bilddaten des ersten Datensatzes mit den Daten des zweiten Datensatzes vorgenommen wird.

8. Verfahren nach einem der vorherigen Ansprüche,
**dadurch gekennzeichnet, dass** bei der Erstellung des zweiten Datensatzes mittels Röntgenscan die Bissschablone (1) von einem Metallblech (6) insbesondere variierender Dicke, umgeben ist.

9. System zur Durchführung des Verfahrens nach einem der vorherigen Ansprüche,
**gekennzeichnet durch,**
ein Kamerasystem zur Aufnahme eines ersten Datensatzes enthaltend dreidimensionale Bilddaten der Oberflächen des zumindest teilweise bezahnten Oberkiefers (13) und des zumindest teilweise bezahnten Unterkiefers (14) des Patienten (9),
eine dem Patienten (9) angepasste Bissschablone (1) mit ausgehärteten Impressionen des Oberkiefers (13) und des Unterkiefers (14) und mit identifizierbaren Markern (4),
ein Aufnahmesystem zur Erstellung eines zweiten Datensatzes dreidimensionaler Röntgendaten enthaltend Oberflächendaten der Bissschablone (1) zur Identifizierung der Oberflächen der Kiefer (13,14) und enthaltend Daten zur Identifizierung der Marker (4),
einen am Unterkiefer des Patienten lösbar befestigbaren Bewegungsaufnehmer (7),
Mittel zur Registrierung der Lage des Bewegungsaufnehmers (7) gegenüber der Bissschablone (1) während der Patient (9) auf die Bissschablone (1) beißt, lösbar am Kopf des Patienten befestigbare Detektormittel (11) zur Aufnahme der Bewegungen des Bewegungsaufnehmers (7) bei Kaubewegungen und zur Speicherung der vom Detektor (11) aufgenommenen Bewegungsdaten in einem dritten Datensatz,
Computermittel zur Vereinigung der dreidimensionalen Bilddaten des ersten Datensatzes mit den Bewegungsdaten des dritten Datensatzes und zur Registrierung vermittels der Daten des zweiten Datensatzes,
einen Bildschirm zur Darstellung der Kaubewegungen des Unterkiefers (14) gegenüber dem Oberkiefer (13).

10. System nach Anspruch 9,
**dadurch gekennzeichnet, dass** die angepasste Bissschablone (1) im vorderen Bereich einen Steckadapter (5) zur mechanischen Verbindung mit dem Aufnahmesystem zur Erstellung eines zweiten Datensatzes, insbesondere mit dem Röntgengerät, aufweist.

## Claims

1. A method for planning treatment on the teeth of a patient (9), the planning being based on a representation of a chewing movement of the patient (9) during which the upper and lower rows of teeth move against each other,
**characterised in that**
a camera is used to record a first data set containing three-dimensional image data of the surfaces of the at least partially toothed upper jaw (13) and of the at least partially toothed lower jaw (14),
the patient (9) is fitted with a bite pattern plate (1), which has cured impressions of the upper jaw (13) and of the lower jaw (14), identifiable markers (4) being provided on and/or in the bite pattern plate (1), a second data set containing three-dimensional image data including surface data of the bite pattern plate (1), in which the surfaces of the jaws (13, 14) and the markers (4) of the bite pattern plate (1) can be identified, is recorded without involving the patient, a movement sensor (7) is releasably fastened to the lower jaw (14) of the patient (9) while the patient is biting on the fitted bite pattern plate (1), the position of the movement sensor (7) relative to the bite pattern plate (1) being registered,
the bite pattern plate is removed,
the movements of the movement sensor (7) during chewing movements are recorded using a detector (11) releasably fastened to the head of the patient (9) and are stored as movement data in a third data set,
the three-dimensional image data of the first data set are combined with the movement data of the third data set, registration taking place by means of the second data set,
the chewing movements of the lower jaw (14) relative to the upper jaw (13) are displayed on a screen for the purpose of treatment planning.

2. The method according to Claim 1,
**characterised in that** the second data set is recorded solely from the bite pattern plate (1).

3. The method according to Claim 1,
**characterised in that** the second data set is produced from a model, in particular a plaster model, of the patient's upper and lower jaws (13, 14), said model being provided with the bite pattern plate (1), the bite pattern plate (1) being inserted between the upper and lower jaws of the model.

4. The method according to any one of the preceding claims,
**characterised in that** the second data set is recorded from the bite pattern plate (1) or the model provided with the bite pattern plate (1) using a three-dimensional X-ray scan.

5. The method according to any one of the preceding claims,
**characterised in that** a holder (8) is provided for the releasable fastening of the movement sensor (7) and is adhesively bonded to the patient's lower jaw.

6. The method according to Claim 5,
**characterised in that** first the movement sensor (7) is fixed to a holder (5) of the bite pattern plate (1) and this first position of the movement sensor (7) is recorded using the detector (11), then the movement sensor (7) is fixed to the adhesively bonded holder (8) and this second position of the movement sensor (7) with the bite pattern plate (1) inserted is recorded using the detector (11), and then the bite pattern plate (1) is removed to allow unimpeded chewing movements.

7. The method according to any one of the preceding claims,
**characterised in that** the three-dimensional image data of the first data set is combined with the movement data of the third data set such that registration of the movement data and the data of the second data set is carried out first by means of the markers (4) of the bite pattern plate (1) which can be recognised therein, and then registration of the image data of the first data set is carried out with the data of the second data set.

8. The method according to any one of the preceding claims,
**characterised in that** when the second data set is created by means of an X-ray scan, the bite pattern plate (1) is surrounded by a metal sheet (6), in particular of varying thickness.

9. A system for carrying out the method according to any one of the preceding claims,
**characterised by**
a camera system for recording a first data set containing three-dimensional image data of the surfaces of the at least partially toothed upper jaw (13) and of the at least partially toothed lower jaw (14) of the patient (9),
a bite pattern plate (1) fitted to the patient (9) and having cured impressions of the upper jaw (13) and of the lower jaw (14) and having identifiable markers (4), a recording system for creating a second data set of three-dimensional X-ray data containing surface data of the bite pattern plate (1) for identifying the surfaces of the jaws (13, 14) and containing data for identifying the markers (4),
a movement sensor (7) which can be releasably fastened to the patient's lower jaw,
means for registering the position of the movement sensor (7) relative to the bite pattern plate (1) while the patient (9) is biting on the bite pattern plate (1),
detector means (11) which can be fastened releasably to the patient's head for recording the movements of the movement sensor (7) during chewing movements and for storing the movement data recorded by the detector (11) in a third data set,
computer means for combining the three-dimensional image data of the first data set with the movement data of the third data set and for registration by means of the data of the second data set,
a screen for displaying the chewing movements of the lower jaw (14) relative to the upper jaw (13).

10. The system according to Claim 9,
**characterised in that** the fitted bite pattern plate (1) has a plug adapter (5) in the front region for mechanical connection to the recording system for creating a second data set, in particular to the X-ray device.

## Revendications

1. Procédé, destiné à planifier un traitement sur la denture d'un patient (9), la planification étant basée sur une représentation d'un mouvement de mastication du patient (9), lors duquel les rangées de dents supérieure et inférieure se déplacent l'une contre l'autre,
**caractérisé**
**en ce qu'**avec une caméra, un premier jeu de données comprenant des données d'images tridimensionnelles de surfaces de la mâchoire supérieure (13) au moins partiellement garnie de dents et de la mâchoire inférieure (14) au moins partiellement garnie de dents est enregistré,
**en ce qu'**on adapte au patient (9) un gabarit d'occlusion (1) qui comporte des impressions solidifiées de la mâchoire supérieure (13) et de la mâchoire inférieure (14), des marqueurs (4) identifiables étant prévus sur et/ou dans le gabarit d'occlusion (1),
**en ce que** sans la participation du patient, on enregistre un deuxième jeu de données avec des données d'images tridimensionnelles comportant des données de surface du gabarit d'occlusion (1), dans lesquelles on peut identifier les surfaces de la mâchoire (13, 14) et les marqueurs (4) du gabarit d'occlusion (1),
**en ce que** sur la mâchoire inférieure (14) du patient (9), on fixe de manière amovible un capteur de mouvement (7), pendant qu'il mord sur le gabarit d'occlusion (1), la position du capteur de mouvement (7) par rapport au gabarit d'occlusion (1) étant consignée,
**en ce qu'**on retire le gabarit d'occlusion,
**en ce que** les mouvements du capteur de mouvement (7) lors de mouvements de mastication sont enregistrés par un détecteur (11) fixé de manière amovible sur la tête du patient (9) et sont mémorisés en tant que données de mouvement dans un troisième jeu de données,
**en ce qu'**on réunit les données d'images tridimensionnelles du premier jeu de données avec les données de mouvement du troisième jeu de données, une consignation étant effectuée au moyen du deuxième jeu de données,
**en ce que** les mouvements de mastication de la mâchoire inférieure (14) par rapport à la mâchoire supérieure (13) sont représentées sur un écran, aux fins de la planification du traitement.

2. Procédé selon la revendication 1,
**caractérisé en ce qu**'on enregistre le deuxième jeu de données du seul gabarit d'occlusion (1).

3. Procédé selon la revendication 1,
**caractérisé en ce qu**'on créé le deuxième jeu de données d'un modèle équipé du gabarit d'occlusion (1), notamment d'un modèle en plâtre pourvu de la mâchoire supérieure et de la mâchoire inférieure (13, 14) du patient, le gabarit d'occlusion (1) étant inséré entre la mâchoire supérieure et la mâchoire inférieure du modèle.

4. Procédé selon l'une quelconque des revendications précédentes,
**caractérisé en ce qu'**on enregistre le deuxième jeu de données du gabarit d'occlusion (1) ou le modèle équipé du gabarit d'occlusion (1) avec un scanner radiographique tridimensionnel.

5. Procédé selon l'une quelconque des revendications précédentes,
**caractérisé en ce qu**'il est prévu un support (8) destiné à la fixation amovible du capteur de mouvement (7), que l'on fixe par collage sur la mâchoire inférieure du patient.

6. Procédé selon la revendication 5,
**caractérisé en ce qu'**on fixe d'abord le capteur de mouvement (7) sur un support (5) du gabarit d'occlusion (1) et on enregistre cette première position du capteur de mouvement (7) à l'aide du détecteur (11), par la suite, le capteur de mouvement (7) étant fixé sur le support (8) collé et cette deuxième position du capteur de mouvement (7) avec le gabarit d'occlusion (1) inséré étant enregistrée à l'aide du détecteur (11), suite à quoi, le gabarit d'occlusion (1) étant retiré, pour permettre des mouvements de mastication non perturbés.

7. Procédé selon l'une quelconque des revendications précédentes,
**caractérisé en ce que** la réunion des données d'images tridimensionnelles du premier jeu de données avec les données de mouvement du troisième jeu de données s'effectue de telle sorte qu'on procède d'abord à une consignation des données de mouvement et des données du deuxième jeu de données au moyen des marqueurs (4) reconnaissables dans celui-ci du gabarit d'occlusion (1) et **en ce qu'**on procède par la suite à une consignation des données d'image du premier jeu de données avec les données du deuxième jeu de données.

8. Procédé selon l'une quelconque des revendications précédentes,
**caractérisé en ce que** lors de l'élaboration du deuxième jeu de données au moyen d'un scanner radiographique, le gabarit d'occlusion (1) est entouré d'une tôle métallique (6), notamment d'épaisseur variable.

9. Système, destiné à réaliser le procédé selon l'une quelconque des revendications précédentes,
**caractérisé par**
un système de caméra, destiné à enregistrer un premier jeu de données comprenant des données d'images tridimensionnelles des surfaces de la mâchoire supérieure (13) au moins partiellement garnie de dents et de la mâchoire inférieure (14) au moins partiellement garnie de dents du patient (9),
un gabarit d'occlusion (1) adapté au patient (9), pourvu d'impressions solidifiées de la mâchoire supérieure (13) et de la mâchoire inférieure (14) et pourvu de marqueurs (4) identifiables,
un système d'enregistrement, destiné à élaborer un deuxième jeu de données de données radiographique tridimensionnelles du gabarit d'occlusion (1), pour identifier les surfaces de la mâchoire (13, 14) et comprenant des données pour l'identification des marqueurs (4),
un capteur de mouvement (7) fixé de manière amovible sur la mâchoire inférieure du patient,
de moyens, destinés à consigner la position du capteur de mouvement (7) par rapport au gabarit d'occlusion (1) pendant que le patient (9) mord sur le gabarit d'occlusion (1),
des moyens de détection (11) fixés de manière amovible sur la tête du patient, destinés à enregistrer les mouvements du capteur de mouvement (7) lors de mouvements de mastication et à mémoriser les données de mouvement enregistrées par le détecteur (11) dans un troisième jeu de données,
des moyens informatiques, destinés à réunir les données d'images tridimensionnelles du premier jeu de données avec les données de mouvement du troisième jeu de données et à les consigner au moyen des données du deuxième jeu de données,
un écran, destiné à représenter les mouvements de mastication de la mâchoire inférieure (14) par rapport à la mâchoire supérieure (13).

10. Système selon la revendication 9,
**caractérisé en ce que** le gabarit d'occlusion (1) adapté comporte dans la zone antérieure un adaptateur enfichable (5) destiné à être relié mécaniquement avec le système d'enregistrement, pour élaborer un deuxième jeu de données, notamment à l'aide de l'appareil radiographique.
